# EUROPEAN PATENT APPLICATION

(11) **EP 2 039 244 A2**
(43) Date of publication of application: **25.03.2009**
(21) Application number: 08020829.1
(22) Date of filing: 16.03.2007
(51) Int. Cl.: A01K 49/00

(54) **Bee nest**

(30) Priority: 17.03.2006 US 783741
(62) Divisional of application: 07734997.5
(71) Applicant: Allan, Matthew, James, Eastleigh Hampshire SO50 9BT (GB); O'Toole, Christopher, Anselm, Leicestershire LE12 7NF (GB)
(72) Inventor: Allan, Matthew, James, Eastleigh Hampshire SO50 9BT (GB); O'Toole, Christopher, Anselm, Leicestershire LE12 7NF (GB)
(74) Representative: Hackett, Sean James

(57) **Abstract**

A bee nest (22) for use in an apparatus (20) for providing solitary bees for pollination comprises a portable on-site incubator (24). The bee nest can comprise a plurality of corrugated sheets (40), each sheet comprising a plurality of flutes. When the sheets are stacked together, the flutes form cavities (44) arranged in a tessellating pattern. The portable incubator (24) can comprise a chamber (26) for holding dormant adult bees, an opening providing access to the chamber, a passage (30) for exiting of bees from the chamber, means (32) for heating the chamber, means (34) for controlling chamber temperature, and a power supply. Dormant adult bees placed in the incubator are stimulated into activity and leave the incubator. Female bees that leave later lay eggs in the bee nest.

## Description

### REFERENCE TO PRIOR APPLICATIONS

This application claims the benefit of U.S. Provisional Application No. 60/783,741, filed on March 17, 2006, which is hereby incorporated by reference.

### FIELD OF INVENTION

The present invention relates generally to bee production, and in particular, to an apparatus and method for providing bees for pollination of a crop.

### RELATED ART

Many crops benefit from pollination by bees, and some are highly dependent on bee pollination. These include the following food crops - almond, apple, avocado, blueberry, canola, cantaloupe, cherry, blueberry, cranberry, cucumber, kiwifruit, nectarine, peach, pear, pepper, plum, prune, raspberry, squash (including pumpkin and gourd), strawberry, sunflower, and tomato; and also crops for seed production, such as alfalfa, asparagus, beet, cabbage and other crucifers, carrot, clover and onion. Crops grown indoors such as in tunnels and glasshouses, and crops grown in very large areas frequently suffer from lack of natural pollinators.

Most commercial pollination is carried out using honeybees. To a lesser extent, particularly in glasshouses, bumblebees *(Bombus* species) are also used as managed pollinators, and in a small number of specialised areas of agriculture some species of solitary bee are utilized. The present invention is directed at improving the utilization of solitary bees as managed pollinators.

Solitary bees have a wide range of nesting habits. Some create cavities in which to nest, by mining or constructing cells; others (among them many species of Megachilid bee, such as *Osmia* and *Megachile* species) search for existing cavities, such as hollow plant stems, beetle borings in timber, pre-used nests created by other insects, and naturally occurring crevices. A mated female bee collects pollen which she packs into the cavity, then lays an egg on the pollen. The cavity is sealed containing one or several eggs which hatch into larvae and eat the stored pollen. Development is completed within the cavity. Eventually, adult bees emerge from the cavity to mate and continue the cycle.

By providing artificial cavities, several species of solitary bee can be encouraged to use them as nests. Artificial nests can be used as trap nests to study existing populations; to improve habitats in order to attract bees or to boost populations; and to manage bees as pollinators in order to produce food crops and seed crops, and enhance pollination of wild flowers.

A number of strategies for artificial nests for solitary bees have been produced that seek to mimic the above-mentioned natural cavities. Nests may be formed from tubes. Hollow plant stems in which bees would naturally nest, such as reeds or bamboo, can be bundled together. These unsophisticated forms of nests are widely known and described, for example in Bosch J and Kemp W, 2001, "How To Manage the Blue Orchard Bee As An Orchard Pollinator", Sustainable Agriculture Network, p20. Instead of using natural cavities provided by hollow plant stems, artificial tubes have also been widely used. These tubes include craft straws or drinking straws, as described in McGregor S E 1976, "Insect Pollination of Cultivated Crop Plants", United States Department of Agriculture, p37, incorporated by reference herein. Thin-walled tubes and stems are vulnerable to parasitoid attack, so the use of thick-walled cardboard tubes has increased.

Nests may alternatively be formed in solid blocks of material, for example, by drilling as described in Cane J, Veirs D and Trostle G, 2003, "How To Build A Nesting Block" USDA-ARS-NPA, Bee Biology And Systematics Laboratory, Logan, Utah, incorporated by reference herein and available on the World Wide Web at loganbeelab.usu.edu. Wood is commonly used as the block material. However, blocks complete with cavities may also be moulded from plastic. Similar to tube nests, additional liners have previously been provided to improve removal and examination of the developing bee.

Nests may also be formed from grooved boards. Nests formed from grooved boards provide significant advantages over the block and tube nests described above since a plurality of cavities in a layer of the stack of boards may be readily accessed by moving an adjacent board of the stack to open the cavity.

The grooves may be formed in a U-shape, which coincides with a flat face of an adjacent board to form a cavity, as described in Bosch J and Kemp W, 2001, "How To Manage the Blue Orchard Bee As An Orchard Pollinator", Sustainable Agriculture Network, incorporated by reference herein.

Alternatively, semi-circular grooves may be formed on both sides of each board of the stack, the semi-circular grooves of adjacent boards being aligned to create an array of cavities having a circular cross-section, as described in U.S. Patent No. 5372535 to Mills, incorporated by reference herein.

In U.S. Patent No. 5618220 to Mills, incorporated by reference herein, there is described a further development of a bee nest in which an array of cavities formed between adjacent boards is tapered at said one end such that said cavities are closed at said one end adjacent the backing material.

For each of the prior art grooved board nests described above, there is a problem in that the number of cavities formed per unit volume of board material is generally low. Thus prior art nests provide a poor yield of bees for the size of the nest. There is therefore a need to improve bee yield. In addition, the weight and volume of grooved board nests require greater labor input to distribute them in the field and subsequently handle them. Thus, there is a need to achieve a bee nest with lower volume and weight than grooved board nests, while retaining the advantage of easy access to view and handle the contents.

In U.S. Patent No. 4319371 to Wiederrich, incorporated by reference herein, there is described an alternative type of nest wherein cavities are formed between alternate layers of flat and corrugated materials wound spirally and contained within a predator and parasite resistant holder. However, the cavities formed in this example have a number of drawbacks. Firstly, access to the cavities is made difficult since the array of cavities are spiral wound and thus the cavities cannot be easily opened up as in the case of a grooved board nest construction. Secondly, the flat and corrugated materials used must necessarily be of a low thickness for the laminated structure to be spiral wound thus subjecting the nesting bees to an increased risk of parasitoid attack. Also, these types of nests are labor-intensive and relatively costly to manufacture.

In US Patent 7086924 to Mills, incorporated by reference herein, there is described further developments of a bee nest. However, the nest requires several different types of components. There is thus a need to achieve a bee nest which is assembled from fewer different types of component, thereby reducing manufacturing costs further and simplifying assembly. In addition, the nest described in US Patent 7086924 is limited in the density of nesting cavities presented to nest-searching females per unit surface area, due to the geometry of the components. Thus, there is a need to achieve a nest which offers a greater density of nesting cavities per unit surface area.

The provision of nest cavities does not of itself guarantee that bees which are released in the vicinity will remain and nest. A proportion of these bees will disperse from the site and reduce the efficiency of the pollination operation. There is a need therefore to improve the attractiveness of the nests to individual bees, most particularly the nesting females.

The flowering of most of the crop plants referred to above takes place over a limited period. In order to achieve effective pollination on a commercial scale it is necessary to ensure that bees are on site and available to forage on the flowers during that period. When honeybees are used, this is a matter of bringing hives with large populations of workers to the site in time. Solitary bees have a different life cycle so their management is different.

In the wild, solitary bees overwinter either as dormant adults or as prepupae. In the case of the former, the dormant adults can be stimulated to break out of their cocoons early by artificially increasing the temperature in incubators, or prevented from emerging until later than normal by dropping the temperature. The use of incubators is well-known in beekeeping for the rearing and maintaining of queen honeybees. To control the emergence of solitary bees so that their flight activity coincides with the flowering of the desired crop the bees may be placed in incubators or chilled cabinets, as described in Bosch J and Kemp W, 2001, "How To Manage the Blue Orchard Bee As An Orchard Pollinator", Sustainable Agriculture Network.

The emergence of certain species of solitary bee, for example *Osmia rufa, O. cornuta* and *O lignaria,* may take place over an extended period, in some cases up to a month. In addition the males start to emerge first, with the females following a few days or a week later. Although the general pattern of emergence is well known, it can be difficult to predict with accuracy what percentages of males and females will have emerged by a certain date under a certain temperature regime. This represents a challenge to the use of these bees as commercial pollinators. In order to ensure that sufficient bees are flying at flowering time, some will be forced to emerge early. These may starve if no other flowers are available, or they may disperse and not return to the crop. On the other hand some bees will emerge too late to do any pollination, in which case the efficiency of the operation is reduced by having to rear unproductive bees.

One conventional approach to providing flying solitary bees on crops to be pollinated is to warm the cocoons until sufficient active adults have emerged. These are chilled again, then transported to the pollination site and released. A drawback with this procedure is the very high dispersal rate that occurs, which requires many more bees being released than are actually necessary to carry out the pollination. Another approach is to warm the cocoons (either by staged temperature increases or directly from chilled temperature to incubation temperature) to a point at which it is calculated the bees are close to emergence, then transport them to the pollination site and allow them to emerge under ambient conditions. A drawback of this procedure is that if temperatures drop the emergence may be delayed for a considerable period, so that the number of flying bees is reduced or even zero at the critical period for pollination. Therefore, there is a need to be able to control the temperature of the bees throughout the emergence period, including the time in the orchard, field or other location where the bees are placed to pollinate a crop.

It is known that bees which are allowed to emerge from loose cocoons placed in the field in containers exhibit high levels of dispersal compared with bees which are permitted to emerge and fly from their natal nests. Thus, there is a need, when providing solitary bees to pollinate a crop, to provide an apparatus in which the bees are placed on site still in their natal nests.

Conventional methods of supplying solitary bees for pollination have incorporated little flexibility, in that once the bees have been moved to the crop, there is no effective way of speeding or slowing the emergence of the bees to respond to the flowering of the crop or ambient conditions. It would be desirable to maintain some control throughout the process.

Utilization of solitary bees and nests in crops requiring pollination has until now required skilled labor to carry out the deployment of nests and the release of bees. Thus, there is a need to simplify these operations.

### SUMMARY

The present invention provides a portable apparatus for providing solitary bees for pollinating a crop. The apparatus can provide a number of active bees at a particular time, and reduce bee dispersal from the crop to be pollinated. The apparatus comprises an incubator and a bee nest, which are preferably deployed as an integral unit or can be deployed on the same site as individual units. Dormant adult bees can be placed in the incubator and heated sufficiently to stimulate the dormant bees into becoming active. In certain embodiments, dormant adult Megachilid bees such as those of the genus *Osmia* or the genus *Megachile* are placed in the apparatus at a site of pollination and stimulated to emerge at a particular time by the application of heat. Emerged female bees can nest in the apparatus after mating so that their offspring can be recovered and utilized for future pollination.

The present invention also provides a nest for solitary bees comprising a plurality of corrugated sheets, each sheet comprising a plurality of flutes, where the sheets stack together to form cavities for bee nesting. Each cavity is formed by combining a flute of one corrugated sheet with a flute of an adjacent stacked sheet. Cavities formed by the plurality of sheets are arranged in a tessellated pattern.

The present invention further provides a portable on-site incubator which can provide for a number of adult bees to emerge from their cocoons on site at a pre-selected time or time interval irrespective of ambient temperature. The portable on-site incubator comprises a thermally insulated chamber in which dormant adult bees can be placed; an opening providing access to the chamber; a passage for exiting of hatched bees from the chamber; means for heating the chamber; means for controlling the temperature within the chamber; and a power supply that provides power to the heating means, the controlling means, or both.

The present invention also provides a method of providing bees, preferably solitary bees of the genus *Osmia* or genus *Megachile,* for pollinating a crop, by providing a portable on-site incubator and dormant adult bees at a site of pollination, heating the dormant bees in the incubator until the bees become active, and then allowing the bees to exit the incubator and disperse over the pollination site. In certain embodiments, dormant adult bees in one or more natal nests are placed within a portable on-site incubator, which then applies heat to stimulate the bees to emerge, preferably in substantial synchrony with the flowering of the crop. Mated females can subsequently lay their eggs in one or more of the natal nests.

Other objects, features and advantages of the present invention will become apparent from the following description when considered in connection with the accompanying figures. It is to be expressly understood, however, that each of the figures is provided for purpose of illustration and description only and is not intended as a definition of the limits of the present invention.

### BRIEF DESCRIPTION OF DRAWINGS

Particular embodiments in accordance with the present invention will now be described with reference to the accompanying drawings in which:
Figure 1 is a cross-sectional view of an integrated portable on-site incubator and bee nest;
Figure 2 is a perspective view of a segment of a pair of corrugated sheets folded to form an array of cavities;
Figure 3 is a perspective view of a bee nest employing a container;
Figure 4 is a perspective view of a bee nest employing a container having ventilation openings;
Figure 5 is a feed compartment for deployment with a bee nest;
Figure 6 is an emergence chamber for deployment with a bee nest;
Figure 7 is a front view of a stack of folded corrugated sheets for a bee nest;
Figure 8 is a cross-sectional view of a pair of folded corrugated sheets showing a flat area adjacent to a fold;
Figure 9 is a cross-sectional view of a portable incubator in which bees are placed as loose cocoons;
Figure 10 is a cross-sectional view of a portable incubator in which bees are placed in an incubator while in their natal nests;
Figure 11 is a cross-sectional view of another embodiment of a portable incubator in which bees are placed in an incubator while in their natal nests;
Figure 12 is a cross-sectional view of a portable incubator having a tunnel extending from a bee exit;
Figure 13 is a cross-sectional view of a bee exiting through a non-return means.
Figure 14 is a cross-sectional partial view of a portable incubator and bee nest showing bees exiting the portable incubator through a tunnel located within and adjacent to the bee nest; and
Figure 15 is a perspective view of an apparatus including a portable on-site incubator and bee nest for providing bees for pollinating a crop.

### DETAILED DESCRIPTION

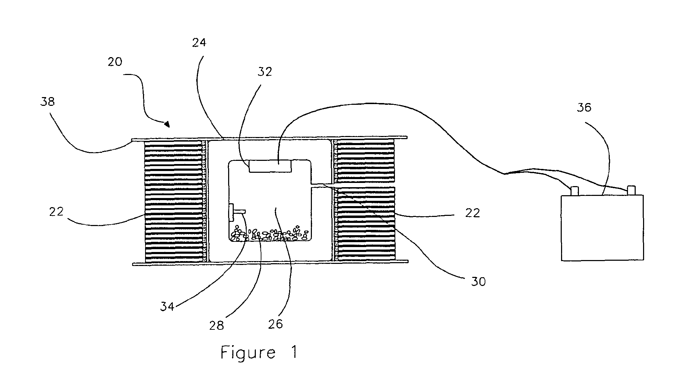

Referring to Fig 1, there is shown an embodiment of the present invention, comprising an apparatus 20 for providing solitary bees, preferably of the genus *Osmia* or the genus *Megachile,* for crop pollination, comprising a bee nest 22 and portable on-site incubator 24 which may be deployed in the field individually or in combination. The bees are placed in the apparatus in a thermally-insulated chamber 26 as dormant adults in cocoons 28 and stimulated to emerge at a selected time for crop pollination by the application of heat. The apparatus also includes means 32 for heating the chamber and means 34 for controlling chamber temperature. Emerged bees can exit the thermally-insulated chamber 26 by a bee exit means 30. The combined nest and portable on-site incubator are housed in a weatherproof housing 38. Female bees can lay their eggs in the apparatus in the bee nest 22 so that their offspring can be recovered and utilized for further pollination. In certain embodiments, more than one bee nest or incubator can be included in the apparatus.

The on-site incubator can provide for a sufficient number of adult solitary bees to emerge from their cocoons and be active at the site of pollination irrespective of the ambient temperature. This can reduce the number of bees used for pollination and enhance the efficiency and profitability of the pollination operation. In addition, by combining a portable incubator with a the bee nest, the pre-nesting dispersal which occurs among female solitary bees can be reduced, as the aroma from and the activity of newly-emerged bees around the exits from the portable on-site incubator can be very attractive to nest-seeking female solitary bees. By reducing pre-nesting dispersal, the number of bees which need to be deployed per unit area of crop can be reduced, further enhancing the efficiency and profitability of the pollination operation. An additional advantage of combining the portable on-site incubator with a bee nest, is that the deployment of bees for pollination can be carried out quickly and easily by unskilled labor.

The means for exiting of bees from the chamber can be one or more tunnels, tubes, channels or other types of passages, or any combination thereof. In cross section, a passage can be circular, U-shaped, square, triangular, trapezoidal or any other geometric shape that allows a bee to exit the chamber.

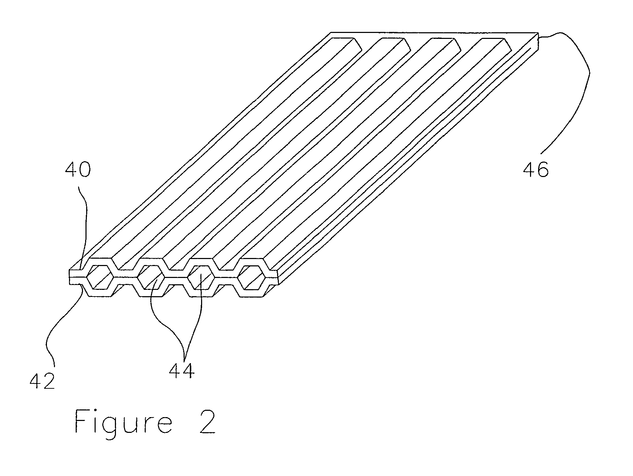

The present invention also provides a bee nest comprising a plurality of corrugated sheets stacked one on top of another. Referring to Figure 2 showing two corrugated sheets of an embodiment, there is shown a single corrugated sheeting folded at its midpoint along line of fold 46 to create a folded pair of corrugated sheets comprising a first corrugated sheet 40 and a second corrugated sheet 42 overlaying first sheet 40 such that flutes from the first sheet 40 are aligned with flutes of the second sheet 42 to form an array of cavities 44. The cavities 44 formed are substantially hexagonal in cross-section. In other embodiments, the cavities can be circular, U-shaped, square, triangular, trapezoidal or any other geometric shape that can support bee nesting. Each corrugated sheeting can be made from waxed cardboard that is biodegradable.

The cavities 44 can have a mean internal cross-sectional diameter of from about 2 mm to about 10 mm, preferably from about 8 mm to about 10 mm, and more preferably approximately 8 mm. The thickness of the part of each corrugated sheet 40, 42 forming the walls of the flutes is preferably greater than the ovipositor length of the major parasitoid wasps (for example *Monodontomerus obscurus)* which attack the species of bee for which the nest is intended. Preferably, flute wall thickness is from about 1 mm to about 3 mm thick, and more preferably, from about 2 mm to about 3 mm thick. Thus, the wall thickness is preferably enough to prevent a female wasp from laying her eggs through the wall of the nest cavity 44 into a cocoon containing a bee. In preferred embodiments, each cavity 44 has a spacing of approximately 18 mm from its adjacent cavity in a planar direction of the sheet. Also, each cavity 44 preferably has a longitudinal length of from about 100 mm to about 200 mm, preferably from about 150 mm to about 180 mm, and more preferably of approximately 150 mm, and each corrugated sheet, when folded, preferably has a longitudinal length of approximately 170 mm.

In other embodiments, each corrugated sheet is formed separately, rather than in pairs. The sheets can be stacked to form a bee nest having tessellating cavities.

In use, access to the interior of cavities 44 in which young bees have been laid can be easily obtained by unfolding the corrugated sheets 40, 42 along line of fold 46, allowing easy examination of young bees in their cocoons for checking health and viability. If parasites or other unsatisfactory conditions are observed, the user can remove parasites and dead or otherwise unsatisfactory bees from the nest cavity 44, or alternatively can remove the satisfactory cocoons and store them separately. The provision of the fold line joining each pair of sheets provides a simple and easily assembled and dismantled cavity construction, and correct alignment of stacked components.

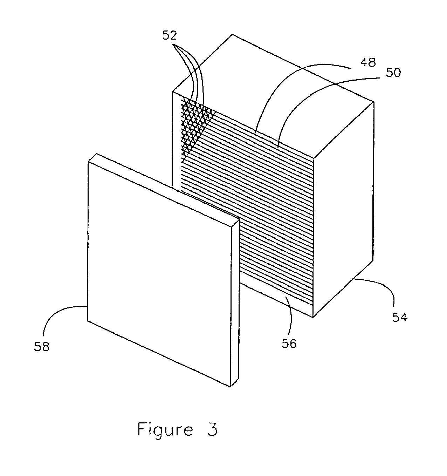

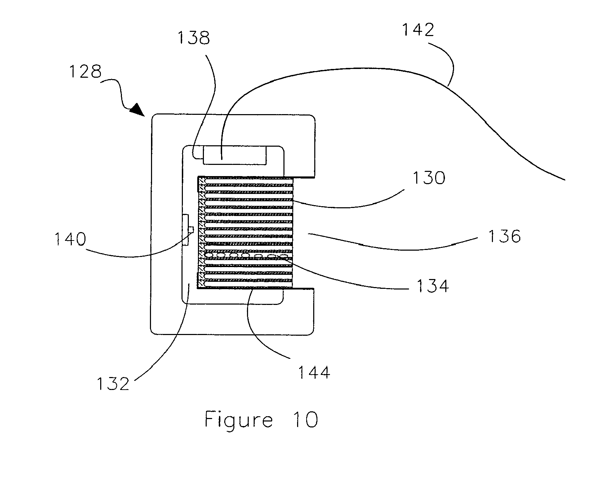

Referring to Figure 3, in one embodiment, each folded pair of corrugated sheets 48, 50 can be stacked with other folded pairs of corrugated sheets in an open container or housing 54. A removable sealing lid 58 can be provided with the housing. The sheets can be stacked such that the fold of each corrugated sheet is placed in the same orientation as the folds of substantially all the other corrugated sheets, i.e., adjacent to one side of the container. For example, Figure 10 shows stacked sheets with folds adjacent to the wall located opposite the opening of the depicted incubator.

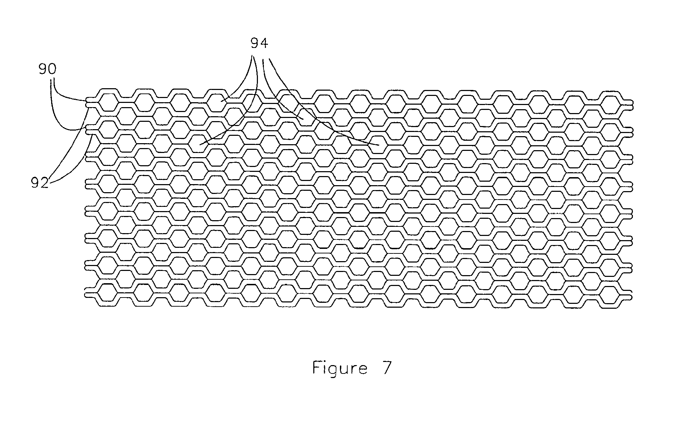

In certain embodiments, at least two, three, or four corrugated sheets, preferably between five (5) and fifty (50) corrugated sheets are stacked. Referring again to Figure 3, since each corrugated sheet can be formed substantially identically, flutes of one corrugated sheet can be aligned above or below flutes of an adjacent corrugated sheet to form the cavities 52. In Figure 3, only a portion of the total number of cavities is shown, for convenience. Nonetheless, cavities are formed throughout the corrugated sheets, as shown for example in Figure 7. In various embodiments, the cavities 52 in an array formed by stacking folded corrugated sheets have substantially similar hexagonal cross-sections that tessellate, thus providing a high ratio of number of cavities to volume of material of the corrugated sheets. In this way the yield of bees per unit volume of the nest can be relatively large.

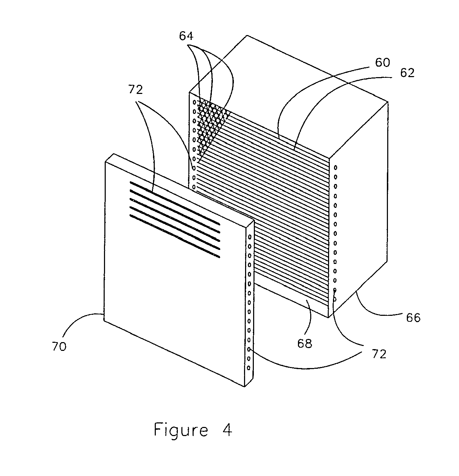

Referring to an embodiment shown in Figure 4 having folded pairs of corrugated sheets 60, 62 forming cavities 64, the container or housing 66 can be provided with a removable sealing lid 70, and ventilation holes 72 can be provided in either the lid, the container or housing, or both. This enables nest components containing live bees to be transported without significant loss of active bees. In practice, nest components containing dormant bees from the previous year can be assembled in the container in advance of deployment in the field without a significant loss of useful bees should they emerge during storage or transport. As in Figure 3, only a portion of the total number of cavities is shown in Figure 4, for convenience.

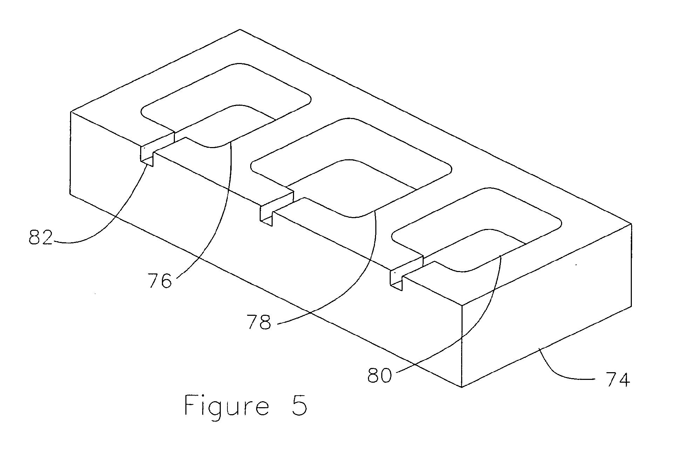

In practice, feed compartments can be provided either attached to or integral with the container or housing, or with the nest. Figure 5 shows an embodiment in which a feed compartment unit 74 includes one or more chambers 76,78,80 in which food and/or water may be provided for the use of emerged adult bees. Each chamber is connected by a short passage to an entrance 82 which enables adult bees within a space formed between the stacked corrugated sheets and the lid of the container or elsewhere to access the food and/or water. Preferably three chambers 76, 78, 80 are provided, in which proteinaceous food, carbohydrate food and water are separately supplied. The unit 74 for providing feed and/or water is optionally inserted in the container in the place of at least one pair of folded corrugated sheets, preferably on top of the stack of folded pairs of corrugated sheets. The feed compartments can provide one or more of the following for bees, especially those which emerge before nectar and pollen become available from the flowers of the crop to be pollinated: (a) water, (b) proteinaceous food, such as natural pollen or a pollen substitute, (c) carbohydrates such as sugar candy or sugar syrup. This provision enables bees to retain their health and strength until the crop flowers.

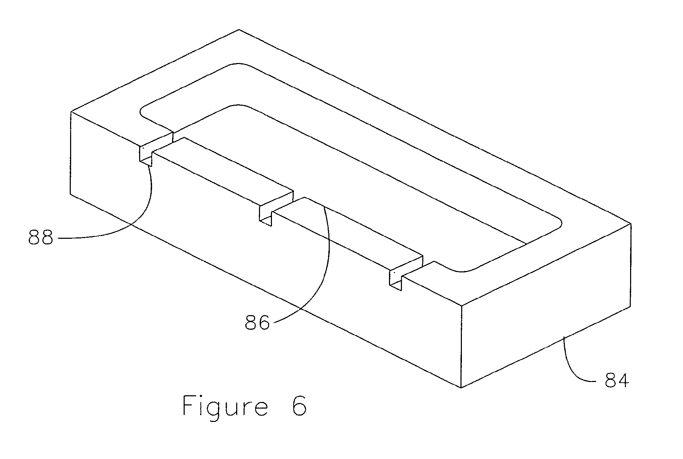

The bee nest can also include an emergence unit for holding cocoons containing dormant adult bees. Referring to an embodiment shown in Figure 6, the emergence unit 84 contains an emergence chamber 86 which is connected by one or a plurality of passages 88 that preferably opens into a space formed between the lid of a bee nest container and the confined bee nest. For example, Figure 3 depicts one embodiment with a space 56 between the lid 58 and the housing 54, and Figure 4 shows another embodiment with a space 68 between the lid 70 and the housing 66. At least one cocoon containing a dormant bee is inserted into the emergence chamber 86. The emergence unit 84 containing the emergence chamber 86 can be inserted in the bee nest container in place of one or more pairs of folded corrugated sheets, preferably between the top of the stack of pairs of folded corrugated sheets and the top inner surface of the container. Adult bees that emerge from cocoons can exit the emergence chamber 86 by the one or plurality of passages 18 and gain access to other parts of the container. Other embodiments are contemplated, including emergence units having a plurality of emergence chambers and/or passages.

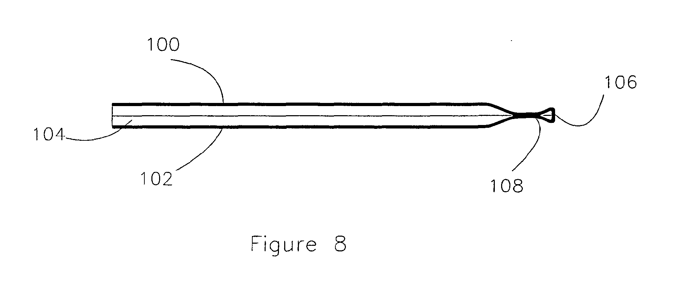

A plurality of stacked folded pairs of corrugated sheets is shown in a front view in Figure 7. Each sheet 90, 92 of a folded pair forms an array of cavities 94. The high density of tessellated cavities is clearly shown. Referring to Figure 8, there is shown a sectional view of an embodiment of a folded pair of corrugated sheets 100 and 102 joined along the line of fold 106. Each sheet preferably incorporates a flat area 108 between the closed ends of the cavities 104 and the line of fold 106. The width of the flat area 108 in the longitudinal direction of the cavities 104 is between about 5 mm and about 25 mm, preferably about 10 mm. By incorporating the flat area 108, stresses which prevent corrugated sheets 100 and 102 touching completely and uniformly can be reduced or substantially eliminated. By providing for corrugated sheets 100 and 102 to touch along the complete length of the cavity 104 when folded, few openings are left for parasites to gain access to bees and food provisions in the cavity 104.

Each corrugated sheet can be formed from wood pulp or paper pulp so as to be disposable and biodegradable. In addition, wood and paper pulp are inexpensive materials which can be formed easily and accurately, thereby providing a nest for solitary bees at a lower cost than prior art nests.

The wood pulp or paper pulp can include, or corrugated sheets can be treated with, a waterproofing agent, such as beeswax, a plant resin, or other agents known in the art. By waterproofing the wood pulp, paper pulp or corrugated sheets, the corrugated sheets can be resistant to mild weathering. In this way, wood or paper pulp-based nest components can have a useful life of more than one season. By incorporating a waterproofing agent, the corrugated sheets can be sterilized by the application of a chemical such as sodium hypochlorite or hydrogen peroxide. By including natural materials as, or in, the waterproofing agent, the wood or paper pulp-based corrugated sheets can remain biodegradable.

Corrugated sheets may also be treated with coloring agents, either incorporated during manufacture or applied post-manufacture. Nesting female bees find it easier and quicker to locate their individual nest in an array of cavities where corrugated sheets are a variety of colors. Dispersal from the nesting site and hence from orchard or field is reduced, thereby improving the efficiency and profitability of the pollination operation. The coloring agents are preferably of low-odor, in order to avoid repelling nesting females.

The wood pulp or paper pulp may include, or corrugated sheets can be treated with, an anti-fungal agent, either integral with the wood pulp or paper pulp, or applied externally after manufacture of the corrugated sheet. The anti-fungal agent may be a fungicide or a fungistat. The action of the anti-fungal agent can reduce mortality among bees caused by fungal growth on pollen stores and on developing and/or adult bees, thereby improving efficiency and profitability of the pollination operation.

A natural or synthetic scent can be applied to the bee nest, preferably an attractant to attract a particular species of bee for which the nest has been provided. The scent may be an aggregation pheromone or an attractant pheromone produced by male or female adult bees, or other scents derived from previously-occupied nest cavities. A synthetic scent can include components of natural scents. By enhancing the attractiveness of the bee nest, drift and dispersal can be reduced, thereby permitting lower numbers of bees to be introduced to a crop, or allowing larger areas of crop to be pollinated using the same number of bees, increasing efficiency and profitability.

In other embodiments, corrugated sheets can be prepared from other materials such as plastic, cardboard, wood, or metal, or any combination of wood pulp, paper pulp, plastic, cardboard, wood and metal. As with wood pulp and paper pulp-based materials, the sheets can incorporate or be treated with a waterproofing agent, a coloring agent, an anti-fungal agent, a natural or synthetic scent, or any combination thereof.

In practice, a liner such as paper straws or a sheet material formed to fit closely within the profile of the flutes of corrugated sheets can be inserted in the cavities. In such an arrangement, the texture of the liner may be more attractive to nest-seeking female bees, the liner can form an additional barrier against some parasites, and the subsequent handling and processing of the bees can be facilitated by the presence of the liner.

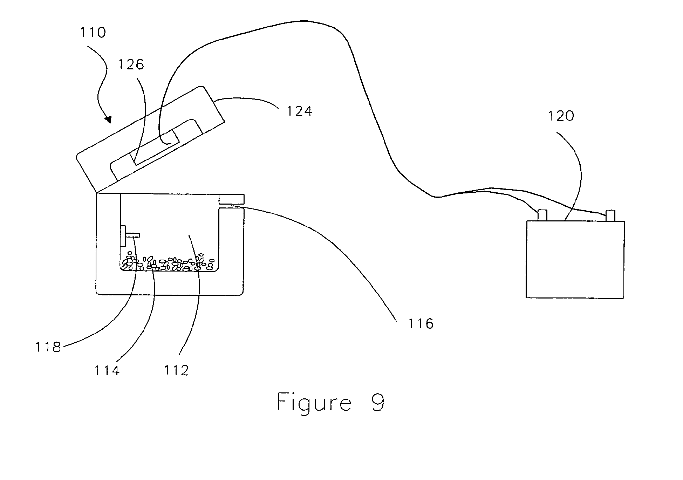

The present invention also provides a portable incubator. Referring to the embodiment in Figure 9, there is shown a portable on-site incubator 110 which can provide for a sufficient number of adult bees to emerge from their cocoons on site at a time or time interval suitable for pollinating a crop, irrespective of ambient temperature. By reducing or substantially eliminating the emergence of bees too early or too late to be effective pollinators, the present invention can provide for a more efficient and profitable pollination system, in which the maximum area of crop can be pollinated with the minimum number of bees.

As shown in Figure 9, an openable lid or door 124 permits access to the interior of a thermally-insulated chamber 112. Dormant adult bees 114 are placed within the thermally-insulated chamber 112 as cocoons. When bees emerge from cocoons under the stimulus of the increased temperature, they are attracted to the daylight and walk out of the portable on-site incubator 110 via an exit passage 116. Means 126 for heating the thermally-insulated chamber 112 is preferably an electrical resistance coil, preferably with a fan to circulate air. In an alternative embodiment, a Peltier thermoelectric device can be used, in which case it is set to operate in heating mode. Other heating devices known in the art are also contemplated. Means 118 for controlling the temperature can be a conventional thermostat, such as an expansion-type thermostat or an electronic thermostat, or other temperature controlling device known in the art. In preferred embodiments, the thermostat is set to maintain the thermally-insulated chamber 112 at a temperature of from about 22°C to about 40°C, preferably about 28°C. In addition, means to modify humidity in the thermally insulated chamber 112, such as a water reservoir with a wick, can be provided.

In the embodiment of Figure 9, a power supply 120 is provided with the portable incubator. The power supply can be included as an integral part of the incubator, or separate from the incubator. The power supply is preferably a rechargeable lead-acid deep cycle battery, although alternative power sources such as other types of battery, line electricity, a wind-powered generator, a passive solar collection device or a solar-powered electricity generator can be used, either singly or in combination. Figure 1 also shows a battery 36 connected to the incubator portion of the portable apparatus. In certain embodiments, two or more power supplies can be included with an incubator. When the power supply 120 is a battery, the energy capacity of the battery is preferably such that the temperature within the thermally-insulated chamber can be held at the selected temperature for a minimum of three days, and more preferably five days, in the ambient climatic conditions typically recorded for the area during the pollination period.

In practice, the volume for the incubator chamber is calculated to accommodate sufficient cocoons to provide an appropriate number of adult females to pollinate the desired area of crop. The desired area to be pollinated by bees from one apparatus is between about 0.4 and about 8 hectares, preferably about 1 hectare. In accordance with the present invention, a number of female cocoons, for example from about 750 to about 2500, and preferably from about 750 to about 1250, can be placed in the portable on-site incubator 54 in order to pollinate a crop covering about 1 hectare. The pollination needs of crops vary, and a suitable number of females will be determined largely empirically in various circumstances. Because females are more significant pollinators than the males, the number of male cocoons is less critical. The proportion of males to females varies between species and in different populations of the same species. In most circumstances the number of males to be placed in the portable on-site incubator can be pro-rata the number of males in the general population.

In preferred embodiments, the volume of the thermally-insulated chamber 112 can be based on a volume of 4 litres per hectare for most crops to be pollinated, and the geometry of the thermally-insulated container can be such that the cocoons are spread in a layer no more than about 35 mm thick, to enable bees to escape readily once they have emerged. Using a deeper layer of cocoons increases the likelihood of meconium being deposited on bees by other bees, with the attendant risk that their wings will be unusable.

In practice, a test incubation can be carried out on a normalized sample of a selected bee population to determine rates of emergence of males and females. The test can be carried out up to four weeks before the expected pollination date. The emergence data can be recorded, and in combination with archive records of flowering of the crop to be pollinated, plus on-site observations of the development of flowers from bud onwards, can be used to calculate the date on which to start incubation and the period of incubation, to provide bees on the crop at the appropriate time. The process can be statistically determined such that a desired proportion of the female bees, preferably over 50%, more preferably 70 to 90%, are actively nesting within a time period, preferably one week, more preferably less than five days, of commencement of crop flowering. Many crops have a short flowering period. For example almonds flower early in the spring, their flowering period is short, and flowering can be subject to adverse weather. In such cases, the more precisely the activity of the bees can be synchronized with the flowering of the almond trees, the better the pollination is likely to be.

Male solitary bees of certain species typically emerge several days earlier than the females, and it may be advantageous in some cases to manipulate the emergence of the different sexes so that their emergence coincides to a greater extent. In a refinement of this procedure, the male and female cocoons can be separated and exposed to different incubation regimes, by varying either or both of the temperature of incubation or the duration of incubation. This may provide for the emergence of both sexes to coincide with the start of flowering.

It is known that agitation of individual cocoons can provoke emergence earlier than undisturbed cocoons. Thus, cocoons can be agitated either prior to being placed in the thermally-insulated chamber or within the thermally-insulated chamber by shaking, by applying pressure, or by other agitating methods, to stimulate early emergence.

To conserve battery power, the incubation process can be started indoors at a facility where bees are processed and/or overwintered, using an alternative power source connected to the portable on-site incubator. When the portable on-site incubator is transported to the crop, a fully-charged battery can be connected on-site, thereby giving the maximum duration of incubation. Where the crop location is a significant distance from the processing and/or overwintering facility, the portable on-site incubator and bee nest can be transported in a heated vehicle. Alternatively, the portable on-site incubator can be powered by a separate power supply within the vehicle in order to conserve battery life for the on-site duty.

Figure 10 illustrates an alternative embodiment of a portable on-site incubator 128 in which the dormant bees 134 are inserted not as loose cocoons, but as cocoons within cells in the nest 130 in which they were laid. The nest 130 is placed on support 144 bodily within the thermally-insulated chamber 132, with the front of the nest 130 aligned towards an opening 136 in the wall of the thermally insulated chamber 132. The opening acts as an exit. When bees emerge, they make their way through cocoons, cell partitions and nest debris toward the exit 136 to the exterior. Support 144 forms a tight fit around nest 130 at the exit 136 in order to reduce heat loss from the thermally-insulated chamber 132.
An electrical heating coil 138 is fitted with a fan to circulate warmed air around nest 130. Temperature control means 140 includes a conventional thermostat. An electrical connection 142 between a power supply and the electrical heating coil 168 is also shown. This embodiment of the portable on-site incubator can confer several advantages. Dispersal is known to be lower when bees emerge from their nests compared with when they are deployed on site as loose cocoons; by utilizing the bees within the nest in which they were laid, and not as loose cocoons, handling can be reduced, processing time can be reduced, and/or potential damage to vulnerable bees can be reduced. In addition, nests from which bees are emerging can be highly attractive to nesting mothers, thereby reducing drift and dispersal, and hence improving efficiency and profitability of the pollination operation.

In practice, the portable incubator can be provided with a device that blocks the exit passage, preventing bees from leaving the chamber when ambient conditions are inhospitable. For example, the device can be a simple removable element or a hinged manually-operated opening element. In certain embodiments, the device can be an automatically opening element which opens after a predefined duration, or when the ambient temperature rises above a predetermined level. In other embodiments, the device can open when solar radiation is sufficient to activate opening, preferably by the action of a bi-metallic strip or the expansion of a fluid in a sealed chamber acting on a lever arm.

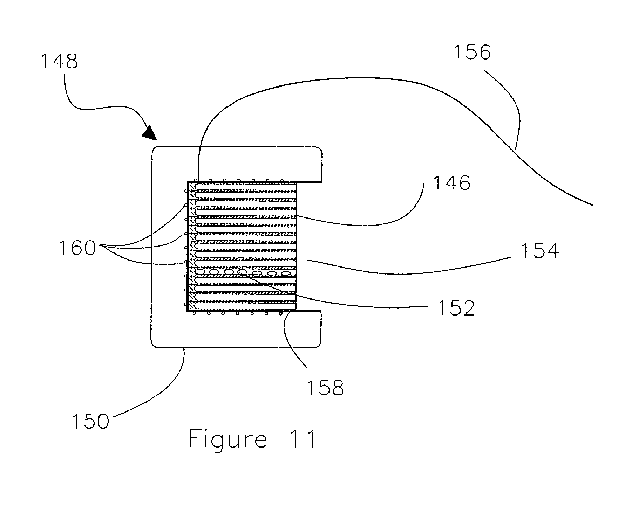

A further embodiment of portable on-site incubator 148 is shown in Figure 11 in which a nest 146 is a sliding fit in a support 158 which maintains close contact with sides, top, bottom and rear of the nest. The support 158 is formed from metal, preferably aluminum, and provides heat to the nest 146 by conduction. The source of heat energy to the support 158 is an electric resistance wire 160 which is adhered to or in close contact with the outer surface of the support 158. In an alternative embodiment, the source of heat energy is a Peltier thermoelectric device operated such that the warm junction is closely coupled to the support 158 in order to conduct heat from the Peltier device to the support 158, and thence to nest 146. An electrical connection 156 between a power supply and the electric resistance wire 160 is also shown.

An advantage of using a Peltier device as the source of heat is that by reversing the battery polarity and hence the current, the Peltier device can act as a cooling device. This enables the portable on-site incubator to keep bees in an overwintering condition, i.e., typically at a temperature of from about 1 to I°c or greater. This can enable the portable on-site incubator to be stocked in advance of crop pollination, while keeping the bees cool. When desired, the Peltier device can be switched to heater mode and the desired incubation process started.

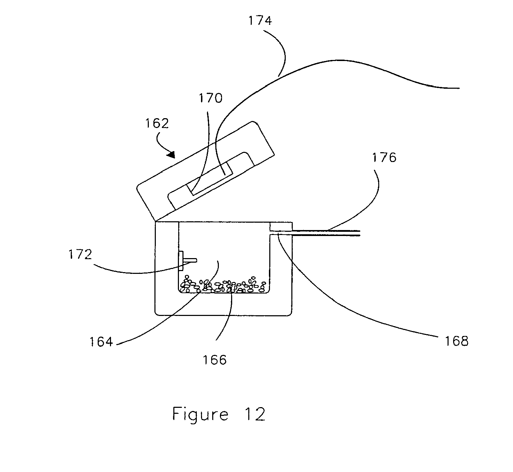

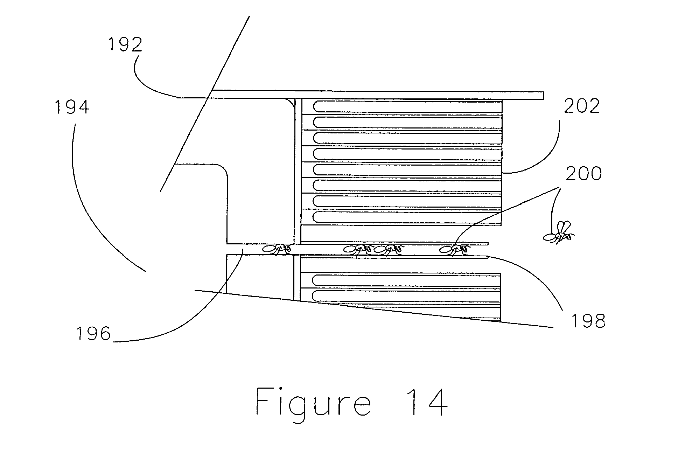

Referring to Figure 12, there is shown an embodiment of a passage or exit 168 from a portable on-site incubator 162. The incubator also comprises a chamber 164, heating means 170, and a thermostat 172. This view of the incubator also shows cocoons 166 in the chamber, and an electrical connection 174 between a power supply and the heating means 170. The exit further comprises one or a plurality of tunnels 176 through which bees have to walk to reach the exterior. The tunnels can be from about 10 mm to about 150mm in length and from about 5 mm to about 10 mm in internal diameter. In certain embodiments, the tunnels are about 25 mm in length and 7.5 mm in internal diameter. The provision of these tunnels 176 can present several benefits. The tunnels 176 mimic the situation bees experience when emerging from their natal nest, thereby making it more likely that emerged bees will remain in the vicinity of the nest; the tunnels 176 greatly reduce air flow into and out of the thermally-insulated chamber 164, thereby diminishing heat loss and extending battery life; the tunnels 176 can be constructed to pass through the bee nest, as depicted in Figure 14.

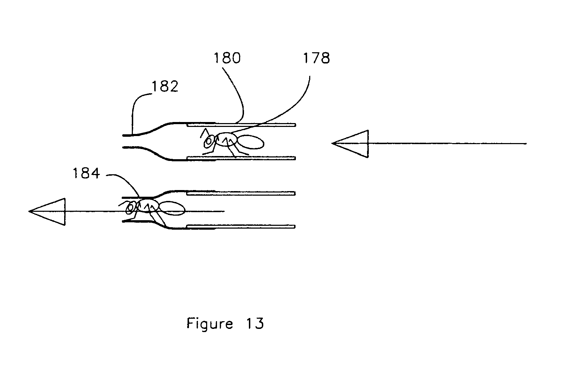

In practice the exit 168, whether including tunnels or not, can be fitted with non-return device in the form of hair, fibre, plastic, sheet or fabric elements which are aligned in such a way that emerging bees can push through the said elements, but not push past them to re-enter the exit 168. One embodiment of such a device is shown in Figure 13. As depicted, a bee 178 in tunnel 180 approaches a closed non-return device 182 and exits by pushing the device into an open position 184. The device returns to a closed position after the bee exits. A non-return device can confer two advantages: (a) the device can prevent nesting mothers from using the exit as a nest to build cells and lay eggs therein, thereby blocking the exit from the thermally-insulated chamber; and (b) the device can reduce heat loss through the exit, thereby increasing battery life.

Figure 14 partially depicts an embodiment of portable on-site incubator 192 where bees 200 are passing from the thermally-insulated chamber 194 through exit passage 196 and tunnel 198. Tunnel 198 is located within bee nest 202. This arrangement reduces dispersal of bees because (a) the tunnels mimic the cavities which bees emerging naturally from their nests experience, (b) the bees are in immediate proximity to nests which are attractive to both males and females, (c) the activity of bees passing through the nests is attractive to nest-seeking females, and (d) the aroma of cocoons is attractive to nest-seeking females.

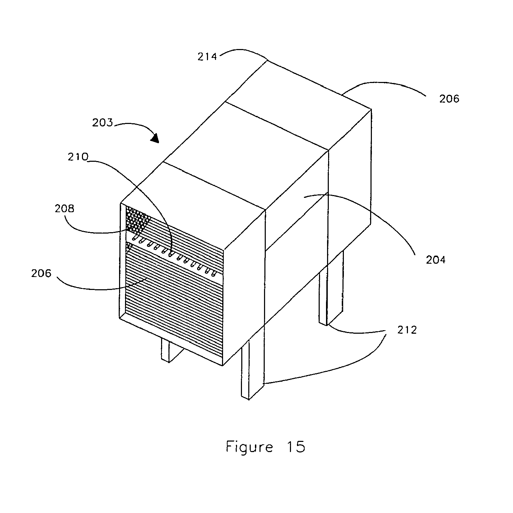

Figure 15 is a perspective view of an apparatus 203 for providing bees for pollinating a crop, showing a portable on-site incubator 204 with bee nests 206 on either side of the incubator. The nest comprises corrugated sheets forming an array of nest cavities 208. In this embodiment, bee exit tunnels 210, which connect to an exit passage from the on-site incubator, are shown. The apparatus is protected by housing 214, which incorporates legs 212 for ease of deployment in field or orchard or other location where a crop is to be pollinated. As with Figures 3 and 4, only a portion of total number of cavities is shown, for convenience.

Efficient utilization of solitary bees for pollination is greater when the emergence of bees coincides with the early stages of flowering of the target crop. If bees emerge too early, they will disperse as no food sources are yet available. If they emerge too late, there will be insufficient time for pollination to be adequately achieved before petal drop. Synchronizing bee emergence and flowering is preferable for a successful pollination operation. Control of the emergence time of bees is known in prior art and can be refined by test incubations with the populations of bees in hand. Prediction of flowering times of target crops can be unreliable, being highly dependent on both long- and short-term climate conditions. Growers' estimates of flowering dates are frequently speculative and acknowledged to be so. In order therefore to improve synchronization of emergence of bees and flowering, it would be advantageous to obtain data from remote orchard and field locations. A further embodiment of the portable on-site incubator therefore includes the provision of sensors such as dataloggers to detect internal and external conditions, such as temperature, humidity, and light levels. In addition cameras can be provided to photograph flowering conditions. The number of bees passing through bee exit means can also be determined. Data collected at the portable on-site incubator can be collated by personnel on site, or can be transmitted by a communication device such as a radio, cellular phone or Internet-based device to a remote operator. The remote operator can have the facility to transmit signals to control the operating functions of the portable on-site incubator in response to the detected conditions. These functions can include temperature within thermally-insulated chamber, on/off switching, and access to the exit 60. Alternatively, the functions of the portable on-site incubator can be automatically controlled in response to the detected data. Not all portable on-site incubators at a location would necessarily be provided with data sensors.

In certain embodiments of the portable apparatus, a nest constructed from the folded corrugated sheets described herein can be included. Alternatively, an apparatus can be prepared using any type of nest that can fit inside or adjacent to the portable incubator.

In a particular embodiment, bees are supplied by providing a portable apparatus comprising a bee nest and a portable incubator, wherein nests containing dormant adult bees in their cocoons within cells constructed by their mothers are placed within the incubator, which then applies heat to stimulate the bees to emerge in synchrony with the flowering of the crop. Emerging bees make their way through cocoons, cell partitions and nest debris to emerge from the apparatus. Nest-seeking females are greatly attracted to the nests because of (a) the odor of previously-used nests, and (b) the activity of newly-emerged bees around the nest. Females rear their young in these previously used nests. At the appropriate time, the apparatus is removed from the orchard or field and put into controlled storage conditions while the bees complete development. When the bees are sufficiently mature to withstand disturbance, the nests are opened and examined for parasites and unhealthy bees. These are either picked out bodily, macerated and removed by vacuum, washed out, or removed by other means. Healthy bees in cocoons are left in the nests, and at selected time, are put into cold storage for winter. When used the following year, the nests are removed from cold store, prepared for emergence, then placed into the portable incubator to provide bees to pollinate a further crop.

Generally, the number of young bees produced during a pollination operation using solitary bees can exceed the number of bees deployed on site to carry out the pollination operation. However, in order to provide suitable numbers of bees for pollination, it may be necessary to augment the populations produced during pollination. In practice, additional bees can be provided by rearing bees in a temporary or permanent enclosure wherein a net, fabric or sheet material is placed over a framework in order to keep bees within the enclosure and parasites out of the enclosure. The framework preferably includes timber or steel members driven or screwed into the ground. Crops are grown within the enclosure to provide food for bees. The additional bees can be released at the site where the portable apparatus containing an incubator is located.

Although the present invention and its advantages have been described in detail, it should be understood that various changes, substitutions and alterations can be made herein without departing from the spirit and scope of the invention. Moreover, the scope of the present application is not intended to be limited to the particular embodiments of the process, manufacture, composition of matter, means, methods and/or steps described in the specification. As one of ordinary skill in the art will readily appreciate from the disclosure of the present invention, processes, manufacture, compositions of matter, means, methods, or steps, presently existing or later to be developed that perform substantially the same function or achieve substantially the same result as the corresponding embodiments described herein may be utilized according to the present invention. Accordingly, the invention is intended to include within its scope such processes, manufacture, compositions of matter, means, methods, or steps.

## Claims

1. A nest for bees, comprising a plurality of corrugated sheets, each sheet comprising a plurality of flutes, wherein the corrugated sheets are stacked together to form cavities, each cavity being formed by combining a flute of one sheet with a flute of a stacked adjacent sheet, and wherein the cavities are arranged in a tessellating pattern.

2. The nest of claim 1, wherein the cavities are substantially circular or hexagonal in cross-section.

3. The nest of claim 1, wherein each corrugated sheet incorporates at least one of an anti-fungal agent, a coloring agent, or a waterproofing agent, or any combination thereof.

4. The nest of claim 1, further comprising natural or synthetic scents for attracting bees.

5. The nest of claim 1, wherein each flute has a wall thickness of from about 1 mm to about 3mm, and each cavity has longitudinal dimension of from about 100mm to about 200mm and a mean internal cross-sectional diameter of from about 2mm to about 10mm.

6. The nest of claim 5, wherein each cavity has a longitudinal dimension of about 150mm and a mean internal cross-sectional diameter of about 8mm.

7. The nest of claim 1, wherein the corrugated sheets are formed in pairs, each pair of sheets being joined together at a fold line perpendicular to the pair's flutes such that when folded, the flutes of one sheet of the pair align with the flutes of the other sheet of the pair to form an array of parallel cavities, each cavity having a closed end adjacent to the fold line.

8. The nest of claim 7, wherein an area of each sheet of the pair adjacent to the fold line is formed as a flat area such that, when the sheets are folded, the flat area of one sheet is in contact with the flat area of the other sheet.

9. The nest of claim 8, wherein the flat area has a width as measured in the longitudinal direction of the cavities of from about 5mm to about 25mm.

10. The nest of claim 9, wherein the width of the flat area is about 10mm.

11. The nest of claim 1, wherein the corrugated sheets comprise wood pulp or paper pulp.
